(19) **Europäisches Patentamt**
**European Patent Office**
**Office européen des brevets**

(11) **EP 4 224 488 B1**

(12) **EUROPEAN PATENT SPECIFICATION**

(45) Date of publication and mention of the grant of the patent:
**11.12.2024   Bulletin 2024/50**

(21) Application number: **22216970.8**

(22) Date of filing: **28.12.2022**

(51) International Patent Classification (IPC):
**G16H 50/20** (2018.01)      **G16H 50/70** (2018.01)
**G16B 20/00** (2019.01)      **G16B 40/30** (2019.01)

(52) Cooperative Patent Classification (CPC):
**G16H 50/20; G16B 20/00; G16B 40/30; G16H 50/70**

(54) **METHOD FOR THE PROGNOSIS OF A DISEASE FOLLOWING UPON A THERAPEUTIC TREATMENT, AND CORRESPONDING SYSTEM AND COMPUTER PROGRAM PRODUCT**

VERFAHREN ZUR PROGNOSE EINER KRANKHEIT NACH EINER THERAPEUTISCHEN BEHANDLUNG SOWIE ENTSPRECHENDES SYSTEM UND COMPUTERPROGRAMMPRODUKT

PROCÉDÉ DE PRONOSTIC D'UNE MALADIE SUITE À UN TRAITEMENT THÉRAPEUTIQUE, SYSTÈME ET PRODUIT PROGRAMME D'ORDINATEUR CORRESPONDANTS

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority:  **02.02.2022   IT 202200001817**

(43) Date of publication of application:
**09.08.2023   Bulletin 2023/32**

(73) Proprietor: **Aizoon S.r.l.**
**10146 Torino (TO) (IT)**

(72) Inventors:
  • **MANGANARO, Lorenzo**
    **10146 TORINO (TO) (IT)**
  • **SABBATINI, Gianmarco**
    **10146 TORINO (TO) (IT)**
  • **BIANCO, Selene**
    **10146 TORINO (TO) (IT)**
  • **CIPOLLINI, Francesca**
    **10146 TORINO (TO) (IT)**
  • **COLOMBI, Davide**
    **10146 TORINO (TO) (IT)**
  • **VATTAKUNNEL, Shaji**
    **10146 TORINO (TO) (IT)**
  • **FALCO, Paolo**
    **10146 TORINO (TO) (IT)**

(74) Representative: **Buzzi, Notaro & Antonielli d'Oulx S.p.A.**
**Corso Vittorio Emanuele II, 6**
**10123 Torino (IT)**

(56) References cited:
**WO-A1-2017/158358**

  • **TONG LI ET AL: "Deep learning based feature-level integration of multi-omics data for breast cancer patients survival analysis", vol. 20, no. 1, 1 December 2020 (2020-12-01), XP055957087, Retrieved from the Internet <URL:https://d-nb.info/1220759872/34> DOI: 10.1186/s12911-020-01225-8**
  • **CHAI HUA ET AL: "Integrating multi-omics data through deep learning for accurate cancer prognosis prediction", BIORXIV, 6 May 2021 (2021-05-06), XP055957085, Retrieved from the Internet <URL:https://www.biorxiv.org/content/10.1101/807214v2.full.pdf> [retrieved on 20220901], DOI: 10.1101/807214**
  • **UNO HAJIME ET AL: "On the C-statistics for evaluating overall adequacy of risk prediction procedures with censored survival data", STATISTICS IN MEDICINE, vol. 30, no. 10, 10 May 2011 (2011-05-10), US, pages 1105 - 1117, XP055957193, ISSN: 0277-6715, DOI: 10.1002/sim.4154**

EP 4 224 488 B1

**Description**

Technical field

The embodiments of the present disclosure relate to techniques for estimating disease-free survival following upon a therapeutic treatment, which may be used for prognosis of the disease.

Background

It is deemed that genetic profiles can be linked to prognosis. Such prognosis is frequently quantified through a quantity that indicates the time of disease-free survival (DFS), typically from cancer, after a particular treatment.

With the invention and recent reduction in cost of next-generation sequencing (NGS) technology, a large amount of *omics* data has become progressively available for biocomputational analyses. In particular, NGS technology is an *in vitro* process of analysis that comprises sequencing in parallel and that enables sequencing of large genomes over a very restricted time. The term "omics" refers to data that identify genomics, transcriptomics, proteomics, metabolomics, and/or metagenomics.

In the last few years, there has thus been a growing interest in the development of machine-learning (ML) models, which are able to decode these correlations, for example for estimating the evolution of the DFS parameter or a similar index as a function of the omics data. For example, in this context it is possible to cite the European patents EP 1 977 237 B1, EP 2 392 678 B1, EP 2 836 837 B1, EP 3 237 638 B1, or EP 2 700 038 B1.

For instance, frequently models that estimate DFS accompany the estimates with a measurement of probability: the decrease in the probability of disease-free survival as a function of time is known as *survival curve.* For example, to estimate disease-free survival time of a patient, the machine-learning model may comprise a parameterized mathematical function, such as an artificial neural network, configured for estimating the time of disease-free survival of a given patient as a function of the omics data obtained for the patient in question, or as a function of a plurality of features drawn from these omics data. Consequently, by acquiring a training dataset that comprises the omics data of a plurality of patients and the respective disease-free survival times, a training algorithm may modify, typically through an iterative process, the parameters of the mathematical function in such a way as to reduce the difference between the estimate of the disease-free survival time and the respective data of the dataset. Consequently, once the learning model has been trained, the mathematical function can provide an estimate of the disease-free survival time for a patient as a function of the respective omics data of the patient.

Instead of directly estimating the DFS, also other parameters indicative of the DFS may be estimated. For example, the article by Tong Li, et al, "Deep learning based feature-level integration of multi-omics data for breast cancer patients survival analysis", vol. 20, no. 1, 1 December 2020 (2020-12-01), XP055957087, DOI: 10.1186/s12911-020-01225-8, discloses a solution wherein a machine learning method, in particular an autoencoder, is used as a feature extraction algorithm in order to generate a set of hidden features. The hidden features are then used to perform a multi-class classification and a survival analysis. Specifically, the survival analysis uses a neural network, which estimates a *"hazard"* based on the hidden features, wherein the neural network is trained with a negative log partial likelihood loss taking into account the survival times of the patients. A similar solution for estimating a hazard/risk score is disclosed in the article by Chai Hua, et al., "Integrating multi-omics data through deep learning for accurate cancer prognosis prediction", bioiv, 6 May 2021 (2021-05-06), XP055957085, D2 DOI:10.1101/807214.

Thus, such a hazard score is in some way indicative of the survival time. For example, the articles by Tong Li and Chai Hua uses a concordance index (C-index) in order to evaluate how well the estimated survival risk/hazard aligns with the actual survival times. In this respect, as also disclosed in document Uno Hajime, et al: "On the C-statistics for evaluating overall adequacy of risk prediction procedures with censored survival data ", STATISTICS IN MEDICINE, vol. 30, no. 10, 10 May 2011 (2011-05-10), pages 1105-1117, XP055957193, US, ISSN: 0277-6715, DOI: 10.1002/sim.4154, such C-statistics are routinely used in the medical literature to assess and quantify the capacity of an estimated risk score in discriminating among subjects with different event times, e.g., via a rank correlation measure.

One of the main problems in this field is that the training dataset typically includes a limited number of samples, especially if compared to the enormous number of features that need to be analyzed for an effective prognosis. In fact, clinical trials typically involve from a few hundreds up to 1500/2000 patients, whereas NGS data may potentially provide as many features as are the human genes: approximately 20 000 in the case of the transcriptome if the analysis is limited to the genes that encode proteins, and a factor of two to three times more if also the non-encoding genes are considered. From the standpoint of machine learning, this problem of dimensionality (generally known as "*curse of dimensionality*") prevents the models from learning, leading to an over-adaptation with respect to the training dataset and to the loss of the capacity of generalization, since there are not enough samples for extrapolating a general behaviour.

Summary

Various embodiments of the present disclosure regard solutions for prognosis, in particular with reference to the estimate of the disease-free survival time following upon a therapeutic treatment. According to one or more embodiments, this object is achieved through a method having the distinctive elements set forth specifically in the ensuing claims. The embodiments moreover regard a corresponding device, as well as a corresponding computer program product that can be loaded into the memory of at least one computer and comprises portions of software code for executing the steps of the method when the product is run on a computer. As used herein, reference to such a computer program product is intended as being equivalent to reference to a computer-readable means containing instructions for controlling a processing system in order to coordinate execution of the process. Reference to "at least one computer" is clearly intended to highlight the possibility of the present disclosure being implemented in a distributed/modular way.

The claims form an integral part of the technical teaching of the description provided herein.

As mentioned previously, various embodiments of the present disclosure regard solutions for prognosis of a disease following upon a therapeutic treatment of the disease, such as a particular type of tumour or cancer. As explained previously, in various embodiments, machine-learning solutions are used for this purpose.

In particular, in various embodiments, during a learning step, a computer receives a dataset comprising a plurality of reference patients who have undergone a therapeutic treatment of the same disease, where the dataset comprises for each reference patient respective omics data and data that indicate a disease-free survival time of the respective reference patient after the respective therapeutic treatment of the disease. For instance, in various embodiments, the omics data comprise transcriptomics data, for example obtained via NGS.

In various embodiments, the computer then generates, for each reference patient, respective pre-processed data, e.g., via an analysis of the principal components of the respective omics data, and stores the respective mapping or combination rules used to generate the pre-processed data as a function of the respective omics data. Optionally, the computer may also normalize the omics data, and/or scale the omics data via a nonlinear function.

In various embodiments, the computer then generates a training dataset to form pairs of reference patients between a respective first reference patient and a respective second reference patient and to determine, for each pair of reference patients, a respective set of features that is calculated via a measurement of distance between the pre-processed data of the respective two reference patients and a respective index that indicates which of the respective two reference patients has a longer disease-free survival time. For instance, for this purpose, the computer may set the index at a first value to indicate the fact that the respective first reference patient has a disease-free survival time that is longer than the disease-free survival time of the respective second reference patient, or else at a second value to indicate the fact that the respective first reference patient has a disease-free survival time that is shorter than the disease-free survival time of the respective second reference patient.

In various embodiments, the computer then trains a classifier configured for estimating the index as a function of a respective set of features that is calculated for the pre-processed data of two patients, using for this purpose the training dataset. For instance, the classifier may comprise at least one of the following: a k-nearest neighbour classifier, an artificial neural network, such as a network of the multilayer-perceptron type, a support vector machine, a Gaussian-process classifier, decision trees, random forests, quadratic discriminant analysis, and/or Gaussian naive Bayes classifiers. In various embodiments, the computer may also select just a subset of the features via a feature-selection method, such as LASSO, and train the classifier in such a way as to estimate the index as a function of the respective subset of features calculated for the pre-processed data of two patients.

Accordingly, compared to the prior art solutions, such as the articles by Tong Li and Chai Hua, the classifier is not trained via the omics data of each individual reference patient, but the input features of the classifier correspond to a distance measurement between the data of two patients. Accordingly, assuming a dataset with $n$ reference patients, the prior-art solutions may use a training dataset with up to $n$ training records, while the solutions disclosed herein may use a training dataset with up to $n(n-1)$ training records, thus significantly increasing the available training data. However, this also implies that the trained classifier does not estimate directly the disease-free survival time (or a similar value such as the hazard or survival risk), but the classifier just estimates an index that indicates which of two patients has a longer disease-free survival time, and a further post-processing is required in order to estimate the disease-free survival time.

Consequently, during a prognosis step, the computer may receive for a (further) patient respective omics data and generate for the patient respective pre-processed data using the mapping or combination rules. In various embodiments, the computer then generates a verification dataset to form pairs of patients by combining the patient with a plurality of reference patients (and preferably with each reference patient) and by determine, for each pair of patients, a respective set of features calculated via the measurement of distance between the pre-processed data of the patient and the pre-processed data of the respective reference patient. Consequently, at this point, the computer may estimate, for each pair of patients, a respective index, supplying to the trained classifier the set of features of the respective pair of patients.

As mentioned before, compared to the prior-art solutions, the classifier is not used to estimate directly the disease-free survival time (or a similar value such as the hazard or survival risk) for the patient based on the omics data of the patient. For example, in the articles by Tong Li and Chai Hua, the classifier provides an individual and absolute risk score for each patient. In this respect, the performance of such prior-art classifiers may be estimated through the C-index values, wherein the C-index represents the fraction of all pairs of individuals whose predicted survival times are correctly ordered based on the estimated hazard, i.e., the C-Index is a performance index of the classifier. Conversely, in the present invention, the classifier estimates for each patient pair an index, which essentially represents a relative score. Accordingly, in the present invention, these indices have to be recalculated for each new patient for a set of reference patients, and just provide a relative information for the pair of patients. However, as will be described in greater detail in the following, the disease-free survival time of the patient may be estimated based on the disease-free survival times of the set of reference patients and the set of estimated indices. Accordingly, also the performance of the present solution could be evaluated by calculating a C-Index for the disease-free survival times estimated by the present solution.

Specifically, in various embodiments, the computer generates in parallel or subsequently a list of the estimated indices, where the list is ordered according to the disease-free survival time of the reference patients of the pairs of patients. In various embodiments, the computer then computes, for each position of a plurality of positions of the list (and preferably for all the positions), a respective parameter that indicates the probability of the patient having a disease-free survival time longer than the disease-free survival time of the reference patient in the respective position, but shorter than the disease-free survival time of the reference patient in the next position. For instance, for this purpose, the computer may generate, for each position, a respective pattern to be verified by selecting the index of the list in the respective position, and a first number of indices of the list before the position, and/or a second number of indices of the list after the position. Then, the computer obtains for each pattern to be verified a respective reference pattern, where the reference pattern corresponds to pattern of indices in the case where the position corresponds to the position where switching occurs between a sequence of the first number of the first value and a sequence of the second number of the second value, and computes the value of the parameter associated to the position via a measurement of similarity or a measurement of distance between the respective pattern to be verified and the respective reference pattern. For instance, in various embodiments, each pattern to be verified corresponds to the sequence of the estimated indices of the list, and the reference pattern associated to a given position has all the indices up to the position set at the first value and all the indices after the position set at the second value.

Consequently, in various embodiments, the computer may select the position for which the respective parameter indicates the highest probability, and estimate the disease-free survival time of the patient as having a value comprised between the disease-free survival time of the reference patient in the selected position and the disease-free survival time of the reference patient in the position that follows the selected position.

Brief description of the drawings

The embodiments of the present disclosure will now be described with reference to the annexed drawings, which are provided purely by way of non-limiting example and in which:

- Figure 1 shows a flowchart of a method and corresponding operation of a computer configured for estimating the disease-free survival time of a patient via a learning step and a prognosis step;
- Figure 2 shows an example of a processing system capable of implementing the operation of Figure 1;
- Figures 3 and 4 show examples of data used in Figure 1;
- Figure 5 shows an embodiment of the learning step of Figure 1;
- Figures 6, 7, and 8 show examples of data used in Figure 5;
- Figure 9 shows an embodiment of the prognosis step of Figure 1;
- Figures 10, 11, 12, and 13 show examples of data used in Figure 9; and
- Figure 14 shows a detail of operation of the embodiment of Figure 9.

Detailed description

In the ensuing description numerous specific details are provided in order to enable an in-depth understanding of the embodiments. The embodiments may be implemented without one or various specific details, or with other methods, components, materials, etc. In other cases, well-known operations, materials, or structures are not represented or described in detail so that the aspects of the embodiments will not be obscured.

Reference throughout the ensuing description to "an embodiment" or "one embodiment" means that a particular characteristic, distinctive element, or structure described with reference to the embodiment is comprised in at least one embodiment. Thus, use of phrases such as "in an embodiment" or "in one embodiment" in various points of this description do not necessarily refer to one and the same embodiment. Moreover, the details, characteristics, distinctive elements, or structures may be combined in any way in one or more embodiments.

The references used herein are provided merely for convenience and do not define the scope or meaning of the embodiments.

As explained before, to enable a more accurate prognosis, recently also omics data are taken in consideration, which are processed by means of a machine-learning algorithm. For instance, in various embodiments, the present solution is used for estimating the disease-free survival time for patients affected by non-small cell lung cancer (NSCLC), who have undergone a surgical operation and have obtained complete removal of the neoplasm. However, the solution proposed herein may be used for estimating the disease-free survival time also for other diseases, and in particular for other types of cancers. For instance, such an estimate may represent auxiliary information fundamental for oncologists, who can make better-informed decisions, for example vary the frequency of the follow-up checks as a function of the time estimated, for instance increase the frequency of the checks for patients with an estimated time that is short.

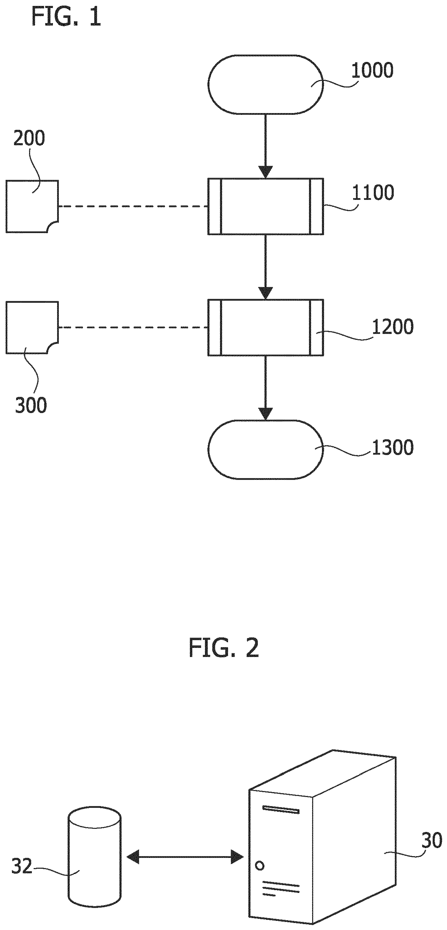

Figure 1 shows an embodiment of operation of a computer 30 configured for estimating the disease-free survival time of a given patient (output datum) as a function of the respective values of the set of parameters (input data).

For instance, as illustrated in Figure 2, the computer 30 may be implemented with any processing system, possibly also in distributed form, and may comprise, for example, a computer, a smartphone or tablet, and/or a remote server. Consequently, operation of the computer 30 may be implemented via software code executed by one or more computers. For instance, in this case, the dataset 200 may be stored in one or more databases 32 managed by the computer 30.

In the embodiment considered, after an initial step 1000, the computer 30 trains, in a step 1100, a machine-learning algorithm using a training dataset 200 that comprises omics data for a plurality of patients. Consequently, in a step 1200, the computer 30 may use the trained algorithm for estimating the disease-free survival time of a patient as a function of the respective omics data 300, and the process terminates in an end step 1300.

As explained previously, the omics data 200 and 300 may comprise *NGS* transcriptomics data (RNA-seq), where the gene expression is expressed, for example, in transcripts per million (TPM). For instance, some examples of these data are made available by *The Cancer Genome Atlas* (TCGA) and may be freely downloaded from many sources, such as https://gdac.broadinstitute.org/ or https://www.cbioportal.org/. For instance, with reference to NSCLC, a dataset 200 may be used that comprises the lung-adenocarcinoma (LUAD) TCGA databases and/or the lung-squamous-cell-carcinoma (LUSC) TCGA databases, which currently represent the main histological subtypes of NSCLC.

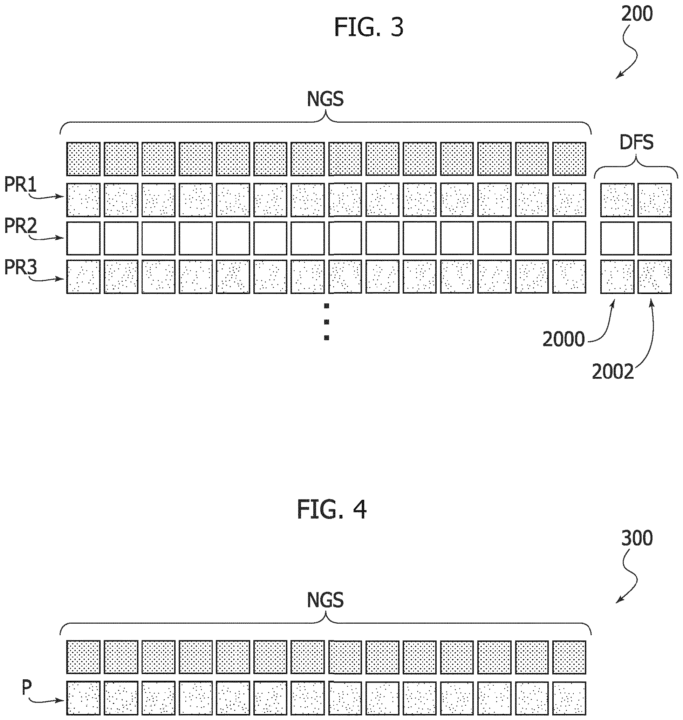

Consequently, as illustrated in Figure 3, the dataset 200 corresponds to a table, list, or matrix of data that comprises the values for a set of variables for a plurality of reference patients PR. In particular, with reference to data *NGS,* each variable corresponds to the gene expression of a particular gene. For instance, the dataset 200 may correspond to a data matrix, in which each row of the matrix represents a (sample) reference patient PR, for example patients $PR_1$, $PR_2$, $PR_3$, etc., and each column represents the gene expression of a particular gene, for example expressed in TPM.

Moreover, each sample/patient of the dataset 200 has, associated thereto, data *DFS* that identify the respective disease-free survival time. For instance, in various embodiments, the disease-free survival time *DFS* of a given patient is identified via a right-censored survival datum, i.e., a pair made up of a Boolean value 2000 and a numeric value 2002, where the Boolean value 2000 indicates whether a relapse of the tumor has been noted for that particular patient (TRUE) or whether, at the date of the last check, no relapse has been noted (FALSE). Moreover, the numeric value 2002 indicates the moment of the relapse in the case where the Boolean is TRUE, or the date of the last check in the case where the Boolean is FALSE. If a patient dies without a tumor for independent reasons, he or she is considered "censored", in which case the Boolean 2000 is set at FALSE, and the numeric value 2002 indicates the moment of death. For instance, this type of coding is used by the TCGA database. However, the datum *DFS* may be identified also via other parameters, and/or a number of parameters, and/or the numeric value 2002 may not identify a date, but directly a period with respect to the date of the operation, for example, a period that has elapsed up to relapse, a period that has elapsed up to the last check, or a period that has elapsed up to death.

Consequently, as shown in Figure 4, the data 300 of a patient P comprise the data *NGS,* and the computer 30 should estimate, in step 1200, a respective disease-free survival time as a function of these data 300.

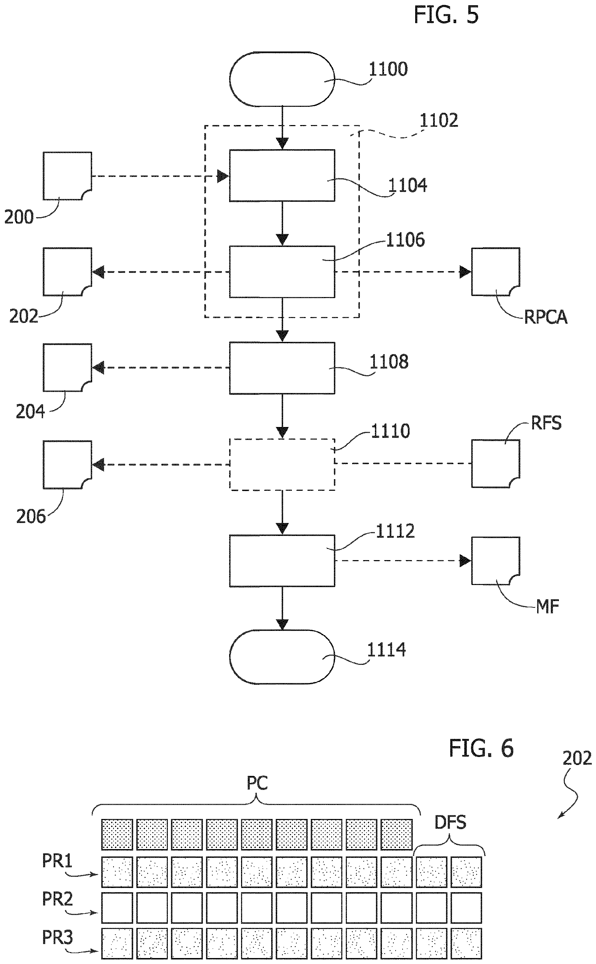

Figure 5 shows a possible embodiment of the learning step 1100. Once the learning step 1100 has been started, the computer 30 obtains the training dataset 200. In various embodiments, the computer 30 then processes, in a step 1102, the data 200. For instance, in various embodiments, the computer 30 carries out an optional pre-processing in a step 1104.

For instance, in various embodiments, the computer 30 may generate data *NGS'* by normalizing, in step 1104, each parameter *NGS* of the dataset 200. In particular, in various embodiments, the computer normalizes the matrix 200 by $10^6$, so that the data *NGS'* of each patient PR, i.e., each row, adds up to 1 instead of $10^6$, as occurs for the standard TPM values.

In various embodiments, the computer 30 may also generate pre-processed data *DFS'* by processing the data *DFS* that indicate a disease-free survival time, for example to converting dates 2002 into a corresponding number of days with respect to the date of treatment.

In addition or as an alternative, the computer 30 may scale, preferably by means of a nonlinear function, the data *NGS* or *NGS'*. For instance, in various embodiments, the computer 30 scales the data *NGS'* with a logarithmic function, such as a binary logarithm (log2).

As explained previously, the matrix 200 (or the matrix resulting from the pre-processing 1104) comprises a number of variables $p$ (e.g., the columns) much higher than the number $n$ of reference patients PR (e.g., the rows), with $n \ll p$. From a mathematical standpoint, the matrix 200 (or the matrix resulting from pre-processing 1104) is hence written in a redundant form, since it can be easily demonstrated that the rank of the columns of the matrix is equal to the rank of the rows. Consequently, in various embodiments, the computer is configured for generating in a step 1106 a matrix 202 by projecting the matrix 200 (or preferably the matrix resulting from pre-processing 1104) into an n-dimensional subspace via a principal-component analysis (PCA). PCA and its variants are well known to the person skilled in the art. For instance, for this purpose, it is possible to cite the book by T. Jolliffe, "Principal Component Analysis", Springer Series in Statistics, Springer-Verlag, New York, 2002, ISBN 0-387-95442-2, the contents of which are incorporated for this purpose herein for reference.

Consequently, as illustrated in Figure 6, step 1106 provides a matrix 202 that comprises only $n$ parameters *PC,* i.e., the so-called principal components. The matrix 202 is hence much smaller than the original one 200, albeit containing the same information, at the cost of having written the new feature $n$ as combination, for example linear combination, of the original features $p$. This is not a problem from a mathematical standpoint, but could reduce the interpretability of the model since it becomes more difficult to set in relation the final result with the genes and understand which genes contribute to the forecasts.

In various embodiments, the computer 30 stores, in step 1106, also the mapping rules RPCA used for generating the $n$ parameters *PC* of a given patient PR as a function of the respective $p$ variables *NGS* (or the respective pre-processed variables), for example the respective function of combination, e.g., linear combination, of the respective $p$ variables.

Consequently, in various embodiments, the dataset 202 comprises a list of $n$ reference samples/patients PR, where each reference element/patient PR of the list comprises $n$ variables *PC* and the respective data *DFS* (or *DFS'*).

In a traditional machine-learning method, such as the solution described in the article by Tong Li, the above dataset 202 (or directly the dataset 200) could then be used to solve a conventional regression problem, in which the model seeks to estimate directly the survival time *DFS* (or a similar value) of a patient, for example using the data *PC* as input of an artificial neural network, where the neural network supplies at output an estimate of the survival time *DFS.*

Instead, in various embodiments of the present disclosure, the machine-learning method receives at input the data of a pair of patients, and the method is trained to estimate which member of the pair has a longer survival time.

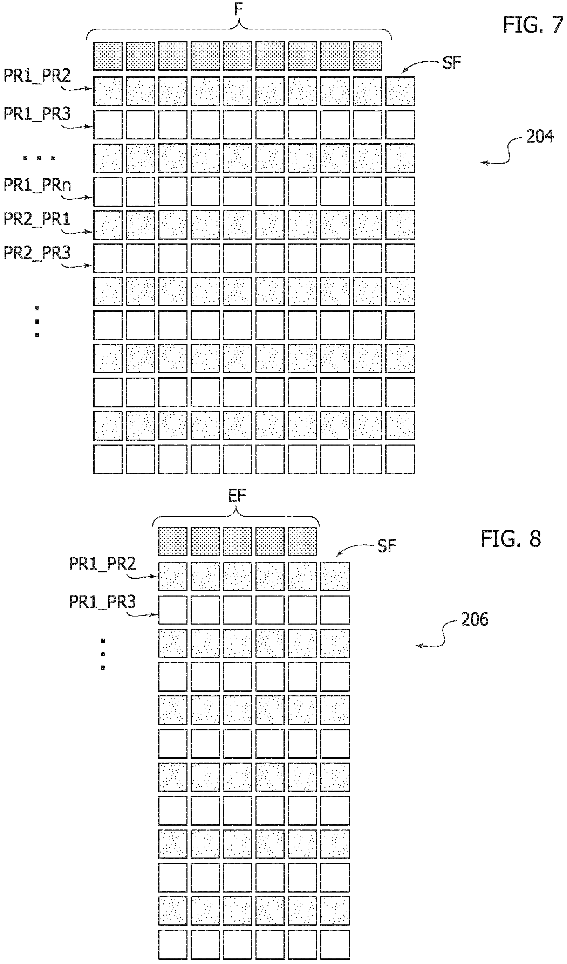

Consequently, in various embodiments, the computer 30 generates, in a step 1108, a list 204, generating pairs of patients by combining all the reference patients PR with one another. For instance, as shown in Figure 7, the computer may generate pairs of patients $PR_1\_PR_2$, $PR_1\_PR_3$, ..., $PR_1\_PR_n$, by combining the first patient $PR_1$ with all the other patients $PR_2,...PR_n$, pairs of patients $PR_2\_PR_1$, $PR_2\_PR_3$, ..., $PR_2\_PR_n$, by combining the second patient $PR_2$ with all the other patients $PR_3,...PR_n$, etc. Hence, in various embodiments, the list 204 comprises $n(n-1)$ elements, denoted hereinafter as elements $PR_x\_PR_y$, where each element $PR_x\_PR_y$ comprises the data of respective features $F$ obtained via the combination of the data *PC* (when the data 202 of step 1106 are used), of the data *NGS'* (when the data of step 1104 are used), or of the data *NGS* (when the original data 200 are used) of a respective first patient $PR_x$ and a respective second patient $PR_y$.

For instance, in various embodiments, the number of the features $F$ corresponds to the number $n$ of the variables/principal components *PC,* and the value of each feature $F_i$, with $1 \le i \le n$, for a given pair of patients $PR_x$ and $PR_y$, i.e., $F_i(PR_x\_PR_y)$, is obtained by combining the value of the respective variable $PC_i$ of the patient $PR_x$, i.e., $PC_i(PR_x)$, with the value of the respective variable $PC_i$ of the patient $PR_y$, i.e., $PC_i(PR_y)$. For instance, in various embodiments, the computer 30 uses a measurement of distance between the values $PC_i(PR_x)$ and $PC_i(PR_y)$. For instance, in various embodiments, the computer is configured for computing the value of each feature $F_i(PR_x\_PR_y)$ via a so-called z-score, where the computer computes the value of the feature $F_i(PR_x\_PR_y)$ as the difference between the respective values $PC_i(PR_x)$ and $PC_i(PR_y)$, for example $F_i(PR_x\_PR_y) = PC_i(PR_x) - PC_i(PR_y)$. Then, the computer 30 computes the standard deviation of the feature $F_i$ and normalizes each value $F_i$ by dividing the value $F_i$ by the respective standard deviation. However, also other measurements of distance may be used, such as Euclidean distance, Minkowski distance, etc.

In various embodiments, the new list/matrix 204 hence comprises $n(n-1)$ elements $PR_x\_PR_y$, with $1 \le x \le n$, $1 \le y < n$ and $x \ne y$, where each element $PR_x\_PR_y$ comprises a set of features $F$, which comprises $n$ features $F_i$, with $1 \le i \le n$, where each feature identifies a measurement of distance between the respective values $PC_i(PR_x)$ and $PC_i(PR_x)$ of the patients $PR_x$ and $PR_y$.

Moreover, the computer 30 determines, for each pair of patients $PR_x\_PR_y$, also a survival index *SF*, which indicates which of the two patients $PR_x$ or $PR_y$ has a longer disease-free survival time, for example by setting the value *SF* at -1 (or 0) in the case where the patient $PR_x$ has a longer disease-free survival time or at +1 in the case where the patient $PR_y$ has a longer disease-free survival time. In particular, for this purpose, the computer 30 may compare the disease-free survival time of the patient $PR_x$, i.e., the data $DFS(PR_x)$ or $DFS'(PR_x)$, with the disease-free survival time of the patient $PR_y$, i.e., the data $DFS(PR_y)$ or $DFS'(PR_y)$.

In this context, it should be noted that the data $DFS$ (or $DFS'$) of two patients $PR_x$ and $PR_y$ should hence be comparable. However, in the case where the dataset also comprises censored patients, not all the pairs $PR_x\_PR_y$ are effectively comparable, since two patients $PR_x$ and $PR_y$ form a comparable pair only when they are both not censored or when only the one who has survived longer is censored. Consequently, in step 1108, the computer 30 may remove these elements, and the final number of elements in the list 204 may even be smaller. Hence, in general, the list 204 may comprise even just a subset of the pairs $PR_x\_PR_y$, where each pair $PR_x\_PR_y$ has associated a respective set of features $F$ determined as a function of the data of the respective patients $PR_x$ and $PR_y$, and a respective index $SF$ that indicates which of the two patients $PR_z$ and $PR_y$ has a longer disease-free survival time.

In various embodiments, after reorganization of the data in step 1108, the computer 30 may then train a machine-learning algorithm in such a way as to estimate the value of the index $SF$ as a function of the values of the features $F$ calculated for two patients. For instance, in various embodiments, the computer 30 may proceed to an optional step 1110 of feature selection, where the computer generates a list 206 that comprises a set of features $EF$, where the computer 30 generates the features $EF$ by selecting a subset of the features $F$; namely, the number of the features $EF$ is smaller than the number of the features $F$, for example smaller than $n$ in the case where the data $PC$ are used (see also Figure 8).

In general, a very large number of feature-selection methods are known. For instance, with reference to the specific application, the inventors have noted that a LASSO model is particularly useful. This method is well known to the person skilled in the art and described, for example, in the article by Tibshirani, Robert, "Regression Shrinkage and Selection via the Lasso", Journal of the Royal Statistical Society, Series B (methodological), 1996, Wiley, 58 (1): 267-88, DOI:10.1111/J.2517-6161.1996.TB02080.X, the contents of which are incorporated herein for reference. For instance, by training a LASSO model in step 1110, the computer 30 may generate a list 206 by removing from the list 204 all the features $F_i$ the LASSO coefficients of which are equal to 0. However, the LASSO method may be replaced with other feature-selection methods, for example one or more methods chosen from the following list:

- wrapper methods, for example using a recursive feature elimination, a forward feature selection, or a backward feature selection;
- filters, for example based upon the chi-squared method, Pearson correlation, relief or Fisher score; or
- embedded methods, for example based upon decision trees, the so-called random forests, sparse multinomial logistic regression, automatic relevance determination, or regularization-based methods, for example ridge and elastic net.

For instance, for this purpose it is possible to cite the article by A. Jovic, *et al. "A review of feature selection methods with applications",* May 2015, 38th International Convention on Information and Communication Technology, Electronics and Microelectronics (MIPRO), DOI: 10.1 109/MIPRO.2015.7160458, the contents of which are incorporated herein for reference.

Consequently, in a step 1112, the computer 30 may train a classifier configured for estimating the value of the survival index $SF$ as a function of the values of the features $EF$ (or even directly of the features $F$) determined for two patients using for this purpose the dataset 206 (or the dataset 204 when step 1110 is omitted).

For instance, in various embodiments the computer 30 uses a k-nearest neighbour (*k*-NN) classifier. However, the computer 30 may also use one or more other classifiers, such as artificial neural networks (for example, a network of the multilayer-perceptron type), support vector machines, Gaussian process classifiers, decision trees, random forests, quadratic discriminant analysis, or Gaussian naive Bayes classifiers. Also these machine-learning methods are well known to the person skilled in the art, and it is possible to cite, for example, Abdullah, Siti, et al. "Support Vector Machine, Multilayer Perceptron Neural Network, Bayes Net and k-Nearest Neighbor in Classifying Gender using Fingerprint Features", International Journal of Computer Science and Information Security, (Vol. 14 No. 7), or Thanh Noi, P., et al. "Comparison of Random Forest, k-Nearest Neighbor, and Support Vector Machine Classifiers for Land Cover Classification Using Sentinel-2 Imagery. Sensors" 2018, 18, 18. DOI:10.3390/s18010018, the contents of which are incorporated herein for reference.

Consequently, by the end of step 1112 the computer 30 has trained the classifier, i.e., determined the respective parameters $MF$ of the classifier, and the learning process terminates in an end step 1114. As explained previously, the specific choice of the algorithm of feature selection 1110 and machine learning 1112 is not particularly important for the purposes of the present disclosure and may also vary according to the specific dataset 200 used and the specific disease considered. In fact, the present description regards above all the reorganization of the dataset 200 into pairs of patients and the estimation of an index $SF$ that indicates which of two patients has a longer disease-free survival time.

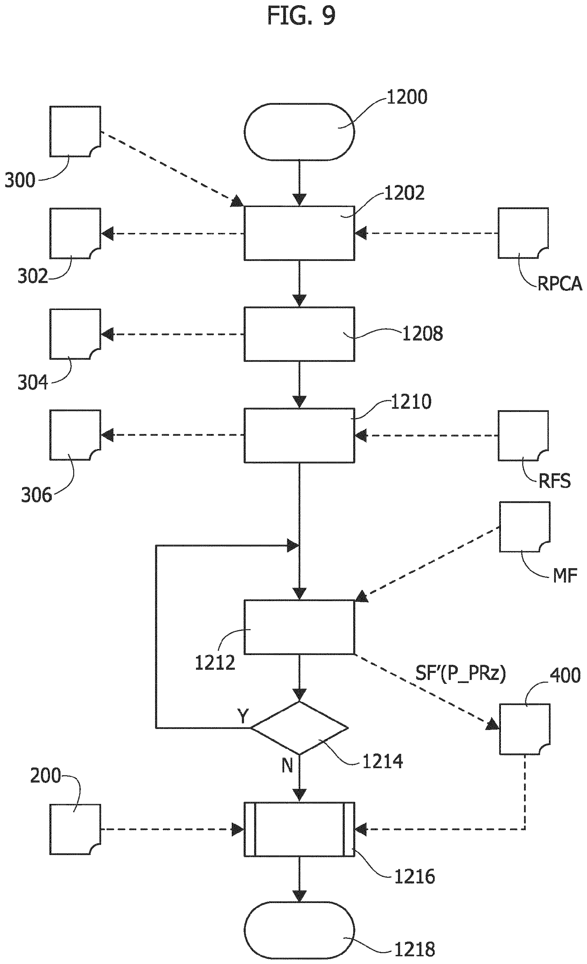

Next, as illustrated in Figure 9, once step 1200 has been started, the computer 30 receives the data 300, in particular the respective data $NGS,$ of a given patient P, and carries out operations similar to the ones carried out in steps 1102-1110.

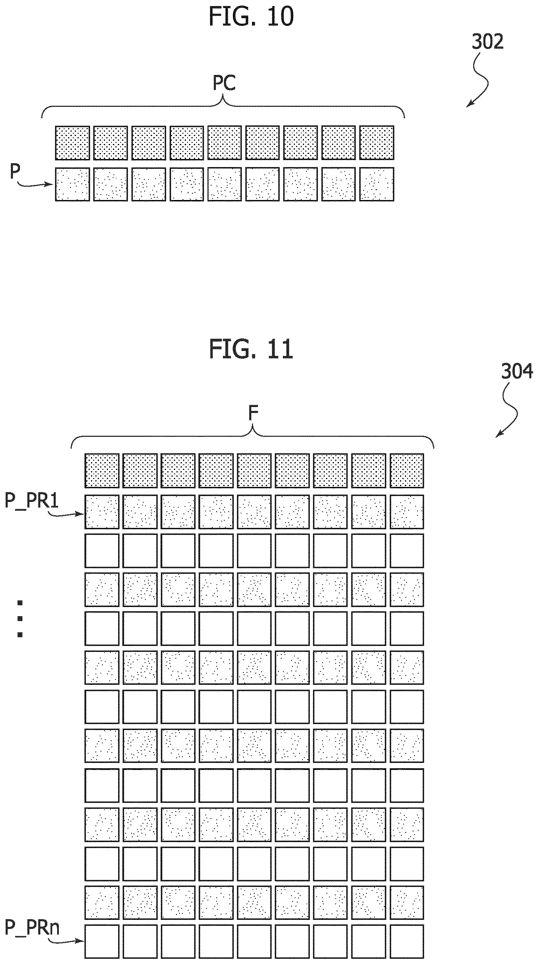

For instance, in various embodiments, the computer 30 may pre-process the data 300, in a step 1202, to generate pre-processed data 302. For instance, as in step 1204, the computer 30 may generate data $NGS'$ by normalizing in step 1104 each parameter $NGS$ of the dataset 300, and/or scale the data $NGS$ or $NGS'$ of the dataset 300. Moreover, in various embodiments, the computer 30 may generate a dataset 302 by computing variables $PC$ as a function of the data $NGS$ (or of the pre-processed data using the mapping rules RPCA generated in step 1106). Consequently, as shown in Figure 10, the dataset 300 of the patient P may be converted into a dataset 302 that comprises $n$ variables $PC$ determined as a function of the data $NGS$.

As explained before, the classifier should receive at input the values determined for pairs of patients. Consequently, in a step 1208, the computer 30 generates a list 304, creating pairs of patients by combining this time the patient P with all the reference patients PR.

For instance, as illustrated in Figure 11, the computer may generate pairs of patients $P\_PR_1$, $P\_PR_2$, ..., $P\_PR_n$. Hence, in various embodiments the list 304 comprises $n$ elements, denoted hereinafter as elements $P\_PR_z$, with $1 \leq z \leq n$, where each element $P\_PR_z$ comprises the data of the respective features $F$ obtained via combination of the data $PC$ (when the data 302 are used), of the data $NGS'$ (when the pre-processed data are used), or of the data $NGS$ (when the original data 300 are used) of the patient P and a respective patient $PR_z$. For instance, in various embodiments, the value of each feature $F_i(P\_PR_y)$ is obtained by combining the value of the respective variable $PC_i$ of the patient P, i.e., $PC_i(P)$, with the value of the respective variable $PC_i$ of the patient $PR_z$, i.e., $PC_i(PR_z)$, for example using the measurement of distance used in step 1108. Consequently, as compared to the list 204, the list 304 does not comprise the index $SF$, but only the values of the features $F$.

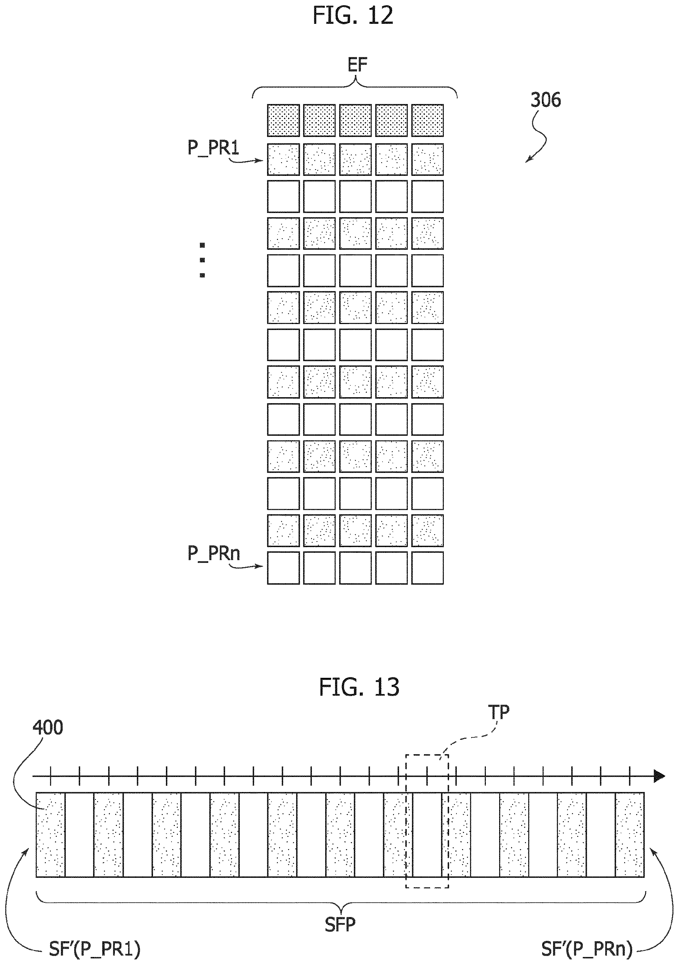

In the case where a feature-selection step 1110 has been used, the computer 30 may then generate, in a step 1210, a list 306 that comprises a subset of features $EF$ by selecting a subset of the features $F$ using the selection rules RFS (see also Figure 12). In general, the computer 30 could also compute directly only the values of the features $EF$.

Consequently, the verification dataset 306 (or directly 304) does not comprise just a single element, but a list of elements, where each element $P\_PR_z$ comprises the respective values of the features $EF$.

In various embodiments, the computer then selects an element $P\_PR_z$ of the list 306 (or 304), for example the element $P\_PR_1$, and uses the trained classifier, i.e., the parameters $MF$, to estimate an index $SF'(P\_PR_z)$ as a function of the respective values of the element selected $P\_PR_z$, and saves the estimated index $SF'(P\_PR_z)$ in a list 400.

The computer 30 may then verify, in a step 1214, whether the list 306 (or directly 304) comprises at least one further element to be classified. In the case where the list 306 (or directly 304) comprises at least one further element (output "Y" from verification step 1214), the computer 30 selects a next element and returns to step 1212 for estimating the respective index $SF'$.

Consequently, via the steps 1212 and 1214, the computer 30 estimates, for each element of the list 304 (or directly 306), a respective index $SF'$, i.e., indices $SF'(P\_PR_1)$, ..., $SF'(P\_PR_n)$ for the elements $P\_PR_1$, ..., $P\_PR_n$. However, these indices $SF'$ provide only an estimate that indicates whether the disease-free survival time of the patient P is longer or shorter than the disease-free survival time of the respective patient $PR_z$.

Consequently, in the case where the computer 30 has processed all the elements in the list, i.e., in the case where the list 306 (or directly 304) does not comprise further elements (output "N" from the verification step 1214), the computer 30 may proceed to a step 1216 where it estimates the disease-free survival time of the patient P as a function of the estimated indices $SF'$ and the disease-free survival times of the reference patients, for example as saved in the dataset 200 (or in the pre-processed dataset 202), and the process terminates in an end step 1218.

Consequently, as illustrated also in Figure 13, step 1216 receives a list 400 that comprises the sequence of the indices $SF'(P\_PR_1)$, ..., $SF'(P\_PR_n)$ for the elements $P\_PR_1$, ..., $P\_PR_n$.

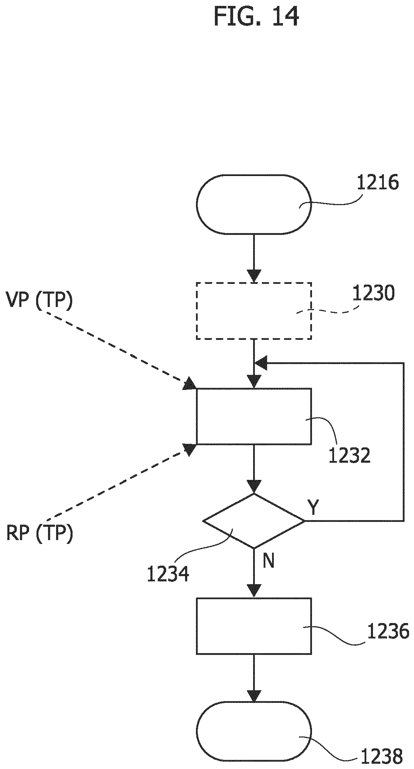

Figure 14 shows a possible embodiment of step 1216.

In particular, once step 1216 has been started, the computer 30 may optionally order, in a step 1230, the list 400 according to the survival time of the reference patients PR of the respective pairs $SF'(P\_PR_1)$, ..., $SF'(P\_PR_n)$. In general, this step is purely optional because the list 306 (or possibly already the list 304) could already be ordered. For instance, in various embodiments, the computer is configured to order, for example in step 1102, the reference patients PR according to their survival time; for example, the patient $PR_1$ may be the patient with the shortest survival time, and the patient $PR_n$ may be the patient with the longest survival time, or vice versa. Consequently, in this way, also the lists 304, 306, and 400 are already ordered.

As a result, in various embodiments, the list 400 comprises a pattern $SFP$ that corresponds to the sequence of the orderly indices $SF'(P\_PR_1)$, ..., $SF'(P\_PR_n)$, where the first index $SFP(1)$ is associated to the reference patient with the shortest survival time (or alternatively the longest survival time), e.g., $SF'(P\_PR_1)$, and the last index $SFP(n)$ is associated to the reference patient with the longest survival time (or alternatively shortest survival time), e.g., $SF'(P\_PR_n)$. For instance, hereinafter it is assumed that each index $SFP/SF'$ is set at a first value, e.g., 0, when the survival time of the patient P is expected to be longer than the survival time of the patient $PR_z$ and is set at a second value, e.g., 1, when the survival time of the patient P is expected to be shorter than the survival time of the patient $PR_z$. For instance, hereinafter it is assumed that the list 400 corresponds to the following pattern *SFP* of orderly indices $SF'(P\_PR_1)$, ..., $SF'(P\_PR_n)$:

$$SFP = \text{"010 0000010 00000110110101110111111110111"}$$

As may be noted, tendentially the classifier is not perfect, and consequently the sequence *SFP* does not comprise a clear separation between a sequence of 0's (first value) and a sequence of 1's (second value), but comprises also reversed values. Consequently, the computer 30 should take into consideration these reversed values.

In particular, in various embodiments, the computer 30 selects a test position *TP,* for example the first position, i.e., *TP* = 1, and computes a congruence index *CI*(*TP*) for the test position *TP.*

For this purpose, in various embodiments, the computer 30 is configured for extracting from the sequence *SFP* for the position *TP* a respective pattern to be verified *VP*(*TP*). For instance, in various embodiments, the pattern *VP*(*TP*) comprises the entire sequence *SFP* of orderly indices $SF'(P\_PR_1)$, ..., $SF'(P\_PR_n)$, i.e., each pattern *VP*(*TP*) comprises the sequence *SFP,* for example:

$$VP(TP) = SFP = \text{"010 0000010 00000110110101110111111110111"}$$

Consequently, in a step 1232, the computer 30 may compare the current pattern *VP*(*TP*) with a reference pattern *RP*(*TP*) that corresponds to the (ideal) sequence of the values *SF'* in the case where the position *TP* corresponds to the position where switching occurs between the sequence of 0's (first value) and the sequence of 1's (second value).

For instance, for the case provided by way of example (*VP*(*TP*) = *SFP*), the patterns *RP*(*TP*) may be the following:

$$- RP(1) = \text{"0111111111111111111111111111111111111111"},$$

$$- RP(2) = \text{"0011111111111111111111111111111111111111"},$$

$$\dots$$

$$- RP(n) = \text{"0000000000000000000000000000000000000000"}.$$

For instance, in various embodiments, the computer 30 determines the congruence index *CI*(*TP*) through a measurement of similarity between the pattern *VP*(*TP*) and the reference pattern *RP*(*TP*), for example by counting the number of values that are the same for the patterns *VP*(*TP*) and *RP*(*TP*), possibly normalizing the results by *n*. Consequently, in this case, the best position corresponds to the position *TP* with the respective highest congruence index *CI*(*TP*). Alternatively, the computer may compute, in step 1232, an (in)congruence index *CI*(*TP*) through a measurement of distance between the pattern *VP*(*TP*) and the reference pattern *RP*(*TP*), for example by computing a sum of the absolute differences *SAD*, possibly normalizing the results by dividing the measurement of distance *SAD* by *n*. Consequently, in this case, the best position corresponds to the position *TP* with the lowest respective congruence index *CI*(*TP*).

Consequently, once the computer 30 has calculated the congruence index *CI*(*TP*) for the current position *TP,* it may verify, in a step 1234, whether at least one further position *TP* has to be verified, for example *TP* < *n*. In the case where at least one further position *TP* has to be verified (output "Y" from the verification step 1234), the computer 30 selects the next position and returns to step 1232.

Instead, in the case where all the positions *TP* have been verified (output "N" from the verification step 1234), the computer 30 proceeds to a step 1236 to select the position *TP\** that corresponds to the best position, i.e., the position *TP* with the lowest (or highest) respective congruence index *CI*(*TP*), and the process terminates in an end step 1238. Consequently, in general, steps 1232 and 1234 may be implemented also directly in steps 1212 and 1214.

For instance, using a normalized congruence index *CI*, the computer 30 could compute the following congruence indices *CI*(*TP*):

$$- CI(1) = 0.575$$

$$- CI(2) = 0.550$$

$$\dots$$

$$- CI(14) = 0.800$$

$$- CI(15) = 0.825,$$

$$- CI(16) = 0.800,$$

$$\dots$$

Consequently, in this case, the computer 30 could select the position $TP^*$ with the congruence index $CI(TP)$ having the maximum value, for example the index $TP = 15$ with $CI(15) = 0.825$. In general, the computer could also take into consideration one or more adjacent congruence (or incongruence) values, for instance computing, in step 1236, a global congruence index $CCI(TP)$ by adding to the congruence index $CI(TP)$ one or more previous and/or subsequent congruence indices; for example,

$$CCI(TP) = CI(TP - 1) + CI(TP) + CI(TP + 1)$$

and then selecting the position $TP^*$ with the respective global congruence index $CCI(TP)$ having the maximum value (or likewise a global incongruence index $CCI(TP)$ having the minimum value). In general, the global index $CCI(TP)$ may thus be obtained by filtering the index $CI(TP)$, for example via a moving average, possibly weighted.

In the embodiment considered, the first pattern $RP(1)$ hence corresponds to the ideal sequence when the patient P has a survival time between that of the first reference patient $PR_1$ and that of the second reference patient $PR_2$. Instead, the last pattern $RP(n)$ corresponds to the ideal sequence when the patient P has a survival time longer than that of the last patient $PR_n$. Consequently, in various embodiments, to verify also the situation in which the patient P has a survival time shorter than that of the first patient $PR_1$, the computer may also verify, in step 1232, a further pattern $RP(0)$ that is compared with the pattern $VP(1)$; for example:

$$- RP(0) = \text{"1111111111111111111111111111111111111111"}$$

In general, the patterns $VP(TP)$ may even correspond just to a subset of the pattern $SFP$. For instance, in various embodiments, each pattern $SFP(TP)$ comprises $k$ orderly indices $SF$, where the parameter $k$ is smaller than, or corresponds to, the number $n$. Preferably, the parameter $k$ is equal for all the sequences $VP$. For instance, in the case where the parameter $k$ is smaller than the number $n$, the parameter $k$ is in any case preferably higher than 10, preferably higher than 50, for example between 100 and 500. Consequently, the pattern $SFP(TP)$ comprises the index $SF'$ at the position $TP$, i.e., $SF'(P\_PR_{TP})$, plus a plurality of indices $SF'$ before and/or after the position $TP$. For instance, by (virtually) adding a sequence of 0's (first value) at the start of the sequence $SFP$ and a sequence of 1's (second value) at the end of the sequence $SFP$, a sequence $VP(TP)$ may be determined by selecting a given number of indices $SF'$ before the position $TP$, the index $SF'$ at the position $TP$, and a given number of indices $SF'$ after the position $TP$, for example always selecting $k = 13$ indices $SF'$:

$$- VP(1) = \text{"0000000100000"}$$

$$- VP(2) = \text{"0000001000000"}$$

$$- VP(3) = \text{"0000010000001"}$$

$$\dots$$

$$- VP(n) = \text{"1111111110111"}$$

Consequently, also in this case, the computer may compute, in step 1232, for each pattern $VP(TP)$ a respective congruence (or incongruence) index $CI(TP)$ by comparing the pattern $VP(TP)$ with a respective pattern $RP(TP)$ that corresponds in this case to the (ideal) sequence of the $k$ values $SF'$ in the case where the position $TP$ corresponds to the position where switching occurs between the sequence of 0's (first value) and the sequence of 1's (second value);

for example:

$$- VP(1) = VP(2) = \ldots = VP(n) = \text{"0 000001111111"}$$

Also in this case, the computer 30 may then select, in step 1236, the position *TP\** with the best index *CI*(*TP*) or *CCI*(*TP*).

In general, the sub-sequences *VP*(*TP*) may be chosen even differently, using, however, the pattern *RP*(*TP*) that corresponds to the (ideal) sequence of the *k* values *SF'* in the case where the position *TP* corresponds to the position where switching occurs between a sequence of 0's (first value) and a sequence of 1's (second value). In fact, it is sufficient for the computer 30 to be able to compute (in step 1232), for each position *TP* of a plurality of positions *TP* of the list 400, a respective parameter *TP* that indicates the probability of the patient P having a disease-free survival time *DFS* longer than the disease-free survival time *DFS* of the reference patient PR in the position *TP*, but shorter than the disease-free survival time *DFS* of the reference patient PR in the next position *TP* + 1.

Consequently, once the computer 30 has selected the position *TP\**, it may estimate the disease-free survival time *DFS*(P) of the patient P with the disease-free survival time *DFS*(PR$_{TP*}$), or in any case a value comprised between the disease-free survival time *DFS*(PR$_{TP*}$) and the disease-free survival time *DFS*(PR$_{TP*+1}$). Consequently, in various embodiments, the computer 30 may determine, for example using the list 200, the survival time of the patient PR at the position *TP\**, i.e., *DFS*(PR$_{TP*}$) and display, for example on a screen of the computer, the time *DFS*(PR$_{TP*}$) as prognosis. Optionally, the computer 30 may also determine, for example using the list 200, the survival time of the patient PR at the position *TP\** + 1, i.e., *DFS*(PR$_{TP*+1}$), and display also this time.

In various embodiments, the computer 30 may determine, in step 1216, also a maximum recommended follow-up time as a function of the time *DFS*(P), for example by dividing the estimated time by a given coefficient, for instance chosen in the range between 1 and 5.

Of course, without prejudice to the underlying principles of the invention, the details of construction and the embodiments may vary widely with respect to what has been described and illustrated herein purely by way of example, without thereby departing from the scope of the present invention as this is defined in the annexed claims.

## Claims

1. A method for prognosis of a given disease following a therapeutic treatment including:

    - during a training phase (1100):

        - receiving a dataset (200) comprising a plurality of reference patients (PR$_1$, PR$_2$, PR$_3$) having performed a therapeutic treatment of said given disease, wherein said dataset (200) comprises for each reference patient (PR) of said plurality of reference patients (PR$_1$, PR$_2$, PR$_3$) respective omics data (*NGS*) and data (*DFS*) indicating a disease-free survival time of the respective reference patient after the respective therapeutic treatment of said given disease;
        - generating (1106) for each reference patient (PR) respective pre-processed data (*PC*) via a principal component analysis of the respective omics data, and storing the respective mapping rules (RPCA) used to generate said pre-processed data (*PC*) as a function of the respective omics data (*NGS*);
        - generating (1108) a training dataset (204) by forming reference patient pairs (PR$_x$_PR$_y$) between a respective first reference patient (PR$_x$) and a respective second reference patient (PR$_y$) and determining for each reference patient pair (PR$_x$_PR$_y$):

            - a respective feature set (*F*) calculated via a distance measure between the pre-processed data (*PC*) of the respective two reference patients (PR$_x$, PR$_y$), and
            - a respective index (*SF*) indicating which of the respective two reference patients (PR$_x$, PR$_y$) has a longer disease-free survival time (*DFS*);

        - training (1110, 1112) a classifier configured to estimate said index (*SF*) as a function of the respective feature set (*F*) calculated for the pre-processed data (*PC*) of two patients by using said training dataset (204); and
        - during a prognosis phase (1200):

            - receiving omics data (*NGS*) of a patient (P);

- generating (1202) for said patient (P) respective pre-processed data (PC) by using said mapping rules (RPCA);
- generating (1208) a verification dataset (304) by forming pairs of patients (P_PR$_z$) by combining said patient (P) with a plurality of reference patients (PR$_z$) and determining for each pair of patients (P_PR$_z$) a respective feature set (F) calculated by said distance measure between the pre-processed data (PC) of said patient (P) and the pre-processed data (PC) of the respective reference patient (PR$_z$);
- estimating (1212) for each pair of patients (P_PR$_z$) a respective index (SF'(P_PR$_z$)) by providing the feature set (F) of the respective pair of patients (P_PR$_z$) to said classifier;
- generating (1212; 1230) a list (400) of said estimated indices (SF'(P_PR$_z$)), wherein said list (400) is sorted according to the disease-free survival time *(DFS)* of the reference patients (PR$_z$) of the pairs of patients (P_PR$_z$);
- calculating (1232) for each position (TP) of a plurality of positions (TP) of said list (400) a respective parameter (CI) indicating the probability that said patient (P) has a disease-free survival time (DFS) being greater than the disease-free survival time (DFS) of the reference patient (PR) in said position (TP), but smaller than the disease-free survival time (DFS) of the reference patient (PR) in the next position (TP), and
- selecting (1236) the position (TP*) for which the respective parameter (CI) indicates the highest probability, and estimating the disease-free survival time (DFS) of said patient with a value between the disease-free survival time (DFS) of the reference patient (PR) in said selected position (TP*) and the disease-free survival time (DFS) of the reference patient (PR) in the position (TP) following said selected position (TP*).

2. The method according to Claim 1, comprising setting said index (SF) to a first value to indicate that the respective first reference patient (PR$_x$) has a disease-free survival time (DFS) being greater than the disease-free survival time (DFS) of the respective second reference patient (PR$_y$), and to a second value to indicate that the respective first reference patient (PR$_x$) has a disease-free survival time (DFS) being smaller than the disease-free survival time (DFS) of the respective second reference patient (PR$_y$).

3. The method according to Claim 2, wherein said calculating (1232) for each position (TP) a respective parameter (CI) comprises:

  - generating for each position (TP) a respective pattern to be verified (VP(TP)) by selecting the index of said list (400) at said position (TP), and a first number of indexes of said list (400) before said position (TP) and/or a second number of indexes of said list (400) after said position (TP),
  - obtaining for each pattern to be verified (VP(TP)) a respective reference pattern (RP(TP)), wherein said reference pattern (RP(TP)) corresponds to a pattern of indices in case said position (TP) corresponds to the position at which occurs the switching between a sequence of said first number of said first value and a sequence of said second number of said second value takes place, and
  - calculating the value of said parameter (CI) associated with said position (TP) by means of a similarity measure or a distance measure between the respective pattern to be verified (VP(TP)) and the respective reference pattern (RP(TP)).

4. The method according to Claim 2 or Claim 3, wherein each pattern to be verified (VP(TP)) corresponds to the sequence of said estimated indices (SF'(P_PR$_z$) of said list (400), and the reference pattern (RP(TP)) associated with said position (TP) has set all indices up to said position (TP) to said first value, and all indices after said position (TP) to said second value.

5. The method according to any of the previous claims, wherein said generating (1106) for each reference patient (PR) respective pre-processed data (PC) comprises normalizing said omics data (DFS), and/or scaling said omics data (DFS) via a non-linear function.

6. The method according to any of the previous claims, wherein said omics data comprises transcriptomic data obtained via Next Generation Sequencing.

7. The method according to any of the previous claims, wherein said training (1110, 1112) a classifier comprises:

  - selecting (1110) a subset (EF) of said features (F) via a feature selection method, such as LASSO, and training a classifier configured to estimate said index (SF) as a function of the respective subset of features (EF) calculated for the pre-processed data (PC) of two patients using said training dataset (204).

8. The method according to any of the previous claims, wherein said classifier comprises at least one of: a k-nearest neighbor classifier, an artificial neural network, such as a multi-layer perceptron type network, a support vector machine, a gaussian process classifier, decision trees, random forests, quadratic discriminant analysis, and/or Naive Bayes gaussian classifiers.

9. A device (400) configured to implement the method according to any of the previous claims.

10. A computer-program product that can be loaded into the memory of at least one processor and comprises portions of software code for implementing the steps of the method according to any of Claims 1 to 8.

**Patentansprüche**

1. Verfahren zur Prognose einer bestimmten Krankheit nach einer therapeutischen Behandlung, aufweisend:

   - während einer Trainingsphase (1100):

      - Empfangen eines Datensatzes (200), der eine Vielzahl von Referenzpatienten ($PR_1$, $PR_2$, $PR_3$) umfasst, die eine therapeutische Behandlung der gegebenen Krankheit durchgeführt haben, wobei der Datensatz (200) für jeden Referenzpatienten (PR) der genannten Vielzahl von Referenzpatienten ($PR_1$, $PR_2$, $PR_3$) entsprechende Omics-Daten (*NGS*) und Daten (*DFS*), die eine krankheitsfreie Überlebenszeit des entsprechenden Referenzpatienten nach der entsprechenden therapeutischen Behandlung der gegebenen Krankheit anzeigen;
      - Erzeugen (1106) von entsprechenden vorverarbeiteten Daten (*PC*) für jeden Referenzpatienten (PR) über eine Hauptkomponentenanalyse der entsprechenden Omics-Daten, und Speichern der entsprechenden Zuordnungsregeln (RPCA), die zum Erzeugen der vorverarbeiteten Daten (*PC*) als eine Funktion der entsprechenden Omics-Daten (*NGS*) verwendet werden;
      - Erzeugen (1108) eines Trainingsdatensatzes (204) durch Bilden von Referenzpatientenpaaren ($PR_x\_PR_y$) zwischen einem entsprechenden ersten Referenzpatienten ($PR_x$) und einem entsprechenden zweiten Referenzpatienten ($PR_y$) und Bestimmen für jede Referenz das Patientenpaar ($PR_x\_ PR_y$):

         - einen entsprechenden Merkmalssatz *(F)*, der über ein Abstandsmaß zwischen den vorverarbeiteten Daten *(PC)* der entsprechenden zwei Referenzpatienten ($PR_x$, $PR_y$) berechnet wird, und
         - einen entsprechenden Index (*SF*), der anzeigt, welche der beiden Referenzpatienten ($PR_x$, $PR_y$) eine längere krankheitsfreie Überlebenszeit (*DFS*) hat;

      - Trainieren (1110, 1112) eines Klassifizierers, der so konfiguriert ist, dass er den Index (*SF*) als eine Funktion des entsprechenden Merkmalsatzes (*F*) abschätzt, der für die vorverarbeiteten Daten (*PC*) von zwei Patienten unter Verwendung des Trainingsdatensatzes (204) berechnet wurde; und
      - während einer Prognosephase (1200):

         - Empfangen von Omics-Daten *(NGS)* eines Patienten (P);

      - Erzeugen (1202) von entsprechenden vorverarbeiteten Daten (*PC*) für den Patienten (P) unter Verwendung der Zuordnungsregeln (RPCA);
      - Erzeugen (1208) eines Verifizierungsdatensatzes (304) durch Bilden von Patientenpaaren ($P\_PR_Z$) durch Kombinieren des Patienten (P) mit einer Vielzahl von Referenzpatienten ($PR_Z$) und Bestimmen eines entsprechenden Merkmalssatzes (*F*) für jedes Patientenpaar ($P\_ PR_Z$), der durch das Abstandsmaß zwischen den vorverarbeiteten Daten (*PC*) des Patienten (P) und den vorverarbeiteten Daten (*PC*) des entsprechenden Referenzpatienten ($PR_Z$) berechnet wurde;

      - Schätzen (1212) eines entsprechenden Index ($SF'(P\_PR_Z)$) für jedes Paar von Patienten ($P\_ PR_Z$) durch Bereitstellen des Merkmalssatzes (*F*) des entsprechenden Paares von Patienten ($P\_ PR_Z$) an den Klassifikator,

      - Erzeugen (1212; 1230) einer Liste (400) der geschätzten Indizes ($SF'(P\_ PR_Z)$), wobei die Liste (400) nach der krankheitsfreien Überlebenszeit (*DFS*) der Referenzpatienten ($PR_Z$) der Patientenpaare ($P\_ PR_Z$) sortiert ist;
      - Berechnen (1232) für jede Position (*TP*) einer Vielzahl von Positionen (*TP*) der Liste (400) eines

entsprechenden Parameter (*CI*), der die Wahrscheinlichkeit angibt, dass der Patient (P) eine krankheitsfreie Überlebenszeit (*DFS*) aufweist, die größer ist als die krankheitsfreie Überlebenszeit (*DFS*) des Referenzpatienten (PR) in der genannten Position (*TP*), aber kleiner als die krankheitsfreie Überlebenszeit (*DFS*) des Referenzpatienten (PR) in der nächsten Position (*TP*), und

- Auswählen (1236) der Position (*TP\**), für die der entsprechenden Parameter (*CI*) die höchste Wahrscheinlichkeit anzeigt, und Schätzen der krankheitsfreien Überlebenszeit (*DFS*) des Patienten mit einem Wert zwischen der krankheitsfreien Überlebenszeit (*DFS*) des Referenzpatienten (PR) in der ausgewählten Position (*TP\**) und der krankheitsfreien Überlebenszeit (*DFS*) des Referenzpatienten (PR) in der Position (*TP*) nach der ausgewählten Position (*TP\**).

2. Verfahren nach Anspruch 1, umfassend das Setzen des Index (*SF*) auf einen ersten Wert, um anzuzeigen, dass der jeweilige erste Referenzpatient ($PR_x$) eine krankheitsfreie Überlebenszeit (*DFS*) aufweist, die größer ist als die krankheitsfreie Überlebenszeit (*DFS*) des entsprechenden zweiten Referenzpatienten ($PR_y$), und auf einen zweiten Wert, um anzuzeigen, dass der jeweilige erste Referenzpatient ($PR_x$) eine krankheitsfreie Überlebenszeit (*DFS*) aufweist, die kleiner ist als die krankheitsfreie Überlebenszeit (*DFS*) des entsprechenden zweiten Referenzpatienten ($PR_y$).

3. Verfahren nach Anspruch 2, wobei das Berechnen (1232) für jede Position (*TP*) einen entsprechenden Parameter (*CI*) umfasst:

   - Erzeugen eines entsprechenden zu überprüfenden Musters (*VP(TP)*) für jede Position (*TP*) durch Auswahl des Index der Liste (400) an der Position (*TP*), und einer ersten Anzahl von Indizes von der Liste (400) vor der Position (*TP*) und/oder eine zweite Anzahl von Indizes der Liste (400) nach der Position (*TP*),
   - Erhalten eines entsprechenden Referenzmusters (*VP(TP)*) für jedes zu überprüfende Muster (*RP(TP)* ), wobei das Referenzmuster (*RP(TP)* ) mit einem Muster von Indizes korrespondiert, wenn die Position (*TP*) mit der Position korrespondiert, an der die Umschaltung zwischen einer Folge der ersten Zahl des ersten Wertes und eine Folge der zweiten Zahl des zweiten Wertes stattfindet, und
   - Berechnung des Wertes des Parameters (*CI*), der mit der Position (*TP*) verknüpft ist, mit Hilfe eines Ähnlichkeitsmaßes oder eines Abstandsmaßes zwischen dem entsprechenden zu überprüfenden Muster (*VP(TP)*) und dem entsprechenden Referenzmuster (*RP(TP)*).

4. Verfahren nach Anspruch 2 oder Anspruch 3, wobei jedes zu überprüfende Muster (*VP(TP)*) mit der Folge der geschätzten Indizes ($SF'(P\_ PR_Z)$) der Liste (400) korrespondiert, und das mit der Position (*TP*) verknüpfte Referenzmuster (*RP(TP)* ) alle Indizes bis zu dieser Position (*TP*) auf den ersten Wert und alle Indizes nach dieser Position (*TP*) auf den zweiten Wert gesetzt hat.

5. Verfahren nach einem der vorhergehenden Ansprüche, wobei das Erzeugen (1106) der entsprechenden vorverarbeiteten Daten (*PC*) für jeden Referenzpatienten (PR) das Normalisieren der Omics-Daten (*DFS*) und/oder das Skalieren der Omics-Daten (*DFS*) über eine nichtlineare Funktion umfasst.

6. Verfahren nach einem der vorhergehenden Ansprüche, wobei die Omics-Daten transkriptomische Daten umfassen, die durch Next Generation Sequencing erhalten wurden.

7. Verfahren nach einem der vorhergehenden Ansprüche, wobei das Training (1110, 1112) einen Klassifikator umfasst:

   - Auswählen (1110) einer Teilmenge (*EF*) der Merkmale (*F*) mit Hilfe eines Merkmalsauswahlverfahrens, wie z.B. LASSO, und Trainieren eines Klassifizierers, der zum Schätzen des Index (*SF*) als Funktion der entsprechenden Untergruppe von Merkmalen (*EF*), die für die vorverarbeiteten Daten (PC) von zwei Patienten unter Verwendung des Trainingsdatensatzes (204) berechnet wurden, konfiguriert ist.

8. Verfahren nach einem der vorhergehenden Ansprüche, wobei der Klassifikator mindestens einen der folgenden Klassifikatoren umfasst: einen K-Nächste-Nachbar-Klassifikator, ein künstliches neuronales Netzwerk, wie z.B. ein mehrschichtiges Perzeptron-Netzwerk, eine Support-Vektor-Maschine, einen Gauß-Prozess-Klassifikator, Entscheidungsbäume, Zufallswälder, eine quadratische Diskriminanzanalyse und/oder Naive Bayes-Gauß-Klassifikatoren.

9. Vorrichtung (400), die so konfiguriert ist, dass sie das Verfahren nach einem der vorhergehenden Ansprüche durchführt.

**10.** Computerprogrammprodukt, das in den Speicher mindestens eines Prozessors geladen werden kann und Teile von Softwarecode zur Implementierung der Schritte des Verfahrens nach einem der Ansprüche 1 bis 8 umfasst.

**Revendications**

**1.** Procédé de pronostic d'une maladie donnée à la suite d'un traitement thérapeutique incluant :

- pendant une phase d'entraînement (1100) :

- la réception d'un ensemble de données (200) comprenant une pluralité de patients de référence ($PR_1$, $PR_2$, $PR_3$) ayant réalisé un traitement thérapeutique de ladite maladie donnée, dans lequel ledit ensemble de données (200) comprend pour chaque patient de référence (PR) de ladite pluralité de patients de référence ($PR_1$, $PR_2$, $PR_3$) une donnée omique (*NGS*) respective et une donnée (*DFS*) indiquant un temps de survie sans maladie du patient de référence respectif après le traitement thérapeutique respectif de ladite maladie donnée ;
- la production (1106) pour chaque patient de référence (PR) d'une donnée prétraitée (*PC*) respective via une analyse en composantes principales de la donnée omique, et le stockage des règles de mappage (RPCA) respectives utilisées pour produire ladite donnée prétraitée (*PC*) en tant que fonction de la donnée omique (*NGS*) respective ;
- la production (1108) d'un ensemble de données d'entraînement (204) en formant des paires de patients de référence ($PR_x\_PR_y$) entre un premier patient de référence ($PR_x$) respectif et un deuxième patient de référence ($PR_y$) respectif et la détermination pour chaque paire de patients de référence ($PR_x\_PR_y$) :

- d'un ensemble de caractéristiques (*F*) respectif calculé via une mesure de distance entre la donnée prétraitée (*PC*) des deux patients de référence ($PR_x$, $PR_y$) respectifs, et
- d'un indice (*SF*) respectif indiquant lequel des deux patients de référence ($PR_x$, $PR_y$) respectifs présente un temps de survie sans maladie (*DFS*) plus long ;

- l'entraînement (1110, 1112) d'un classificateur configuré pour estimer ledit indice (*SF*) en tant que fonction de l'ensemble de caractéristiques (*F*) respectif calculé pour la donnée prétraitée (*PC*) de deux patients à l'aide dudit ensemble de données d'entraînement (204) ; et
- pendant une phase de pronostic (1200) :

- la réception d'une donnée omique (*NGS*) d'un patient (P) ;

- la production (1202) pour ledit patient (P) d'une donnée prétraitée (*PC*) respective à l'aide desdites règles de mappage (RPCA) ;
- la production (1208) d'un ensemble de données de vérification (304) en formant des paires de patients ($P\_PR_z$) en combinant ledit patient (P) avec une pluralité de patients de référence ($PR_z$) et la détermination pour chaque paire de patients ($P\_PR_z$) d'un ensemble de caractéristiques (*F*) respectif calculé par ladite mesure de distance entre la donnée prétraitée (*PC*) dudit patient et la donnée prétraitée (*PC*) du patient de référence ($PR_z$) respectif ;
- l'estimation (1212) pour chaque paire de patients ($P\_PR_z$) d'un indice ($SF'(P\_PR_z)$) respectif en fournissant l'ensemble de caractéristiques (*F*) de la paire de patients ($P\_PR_z$) respective audit classificateur ;
- la production (1212 ; 1230) d'une liste (400) desdits indices ($SF'(P\_PR_z)$) estimés, dans lequel ladite liste (400) est triée en fonction du temps de survie sans maladie (*DFS*) des patients de référence ($PR_z$) des paires de patients ($P\_PR_z$) ;
- le calcul (1232) pour chaque position (*TP*) d'une pluralité de positions (*TP*) de ladite liste (400) d'un paramètre (*CI*) respectif indiquant la probabilité que ledit patient (P) présente un temps de survie sans maladie (*DFS*) qui est plus long que le temps de survie sans maladie (*DFS*) du patient de référence (PR) dans ladite position (*TP*), mais plus court que le temps de survie sans maladie (*DFS*) du patient de référence (PR) dans la position (*TP*) suivante, et
- la sélection (1236) de la position (*TP**) pour laquelle le paramètre (*CI*) respectif indique la probabilité la plus haute, et l'estimation du temps de survie sans maladie (*DFS*) dudit patient avec une valeur entre le temps de survie sans maladie (*DFS*) du patient de référence (PR) dans ladite position (*TP**) sélectionnée et le temps de survie sans maladie (*DFS*) du patient de référence (PR) dans la position (*TP*) suivant ladite position (*TP**) sélectionnée.

**2.** Procédé selon la revendication 1, comprenant le réglage dudit indice (*SF*) à une première valeur pour indiquer que le premier patient de référence ($PR_x$) respectif présente un temps de survie sans maladie (*DFS*) qui est plus long que le temps de survie sans maladie (*DFS*) du deuxième patient de référence ($PR_y$) respectif, et à une deuxième valeur pour indiquer que le premier patient de référence ($PR_x$) respectif présente un temps de survie sans maladie (*DFS*) qui est plus court que le temps de survie sans maladie (*DFS*) du deuxième patient de référence ($PR_y$) respectif.

**3.** Procédé selon la revendication 2, dans lequel ledit calcul (1232) pour chaque position (*TP*) d'un paramètre (*CI*) respectif comprend :

- la production pour chaque position (*TP*) d'un motif à vérifier (*VP(TP)*) respectif par la sélection de l'indice de ladite liste (400) en ladite position (*TP*), et d'un premier nombre d'indices de ladite liste (400) avant ladite position (*TP*) et/ou d'un deuxième nombre d'indices de ladite liste (400) après ladite position (*TP*),
- l'obtention pour chaque motif à vérifier (*VP(TP)*) d'un motif de référence (*RP(TP)*) respectif, dans lequel ledit motif de référence (*RP(TP)*) correspond à un motif d'indices dans le cas où ladite position (*TP*) correspond à la position à laquelle survient la commutation entre une séquence dudit premier nombre de ladite première valeur et une séquence dudit deuxième nombre de ladite deuxième valeur, et
- le calcul de la valeur dudit paramètre (*CI*) associé à ladite position (*TP*) au moyen d'une mesure de similarité ou d'une mesure de distance entre le motif à vérifier (*VP(TP)*) respectif et le motif de référence (*RP(TP)*) respectif.

**4.** Procédé selon la revendication 2 ou la revendication 3, dans lequel chaque motif à vérifier (*VP(TP)*) correspond à la séquence desdits indices estimés ($SF'(P\_PR_z)$) de ladite liste (400), et le motif de référence (*RP(TP)*) associé à ladite position (*TP*) a réglé tous les indices jusqu'à ladite position (*TP*) à ladite première valeur, et tous les indices après ladite position (*TP*) à ladite deuxième valeur.

**5.** Procédé selon l'une quelconque des revendications précédentes, dans lequel ladite production (1106) pour chaque patient de référence (*PR*) d'une donnée prétraitée (*PC*) respective comprend la normalisation de ladite donnée omique (*DFS*), et/ou la mise à l'échelle de ladite donnée omique (*DFS*) via une fonction non linéaire.

**6.** Procédé selon l'une quelconque des revendications précédentes, dans lequel ladite donnée omique comprend une donnée transcriptomique obtenue via un Séquençage de Nouvelle Génération.

**7.** Procédé selon l'une quelconque des revendications précédentes, dans lequel ledit entraînement (1110, 1112) d'un classificateur comprend :

- la sélection d'un sous-ensemble (*EF*) desdites caractéristiques (*F*) via un procédé de sélection de caractéristiques, tel un LASSO, et l'entraînement d'un classificateur configuré pour estimer ledit indice (*SF*) en tant que fonction du sous-ensemble de caractéristiques (*EF*) respectif calculé pour la donnée prétraitée (*PC*) de deux patients à l'aide dudit ensemble de données d'entraînement (204).

**8.** Procédé selon l'une quelconque des revendications précédentes, dans lequel ledit classificateur comprend au moins l'un parmi : un classificateur k du plus proche voisin, un réseau neuronal artificiel, tel un réseau de type perceptron multicouche, un séparateur à vaste marge, un classificateur à processus gaussien, des arbres de décision, des forêts aléatoires, une analyse à discrimination quadratique et/ou des classificateurs gaussiens Bayes Naïfs.

**9.** Dispositif (400) configuré pour mettre en oeuvre le procédé selon l'une quelconque des revendications précédentes.

**10.** Produit de programme informatique qui peut être chargé dans la mémoire d'au moins un processeur et qui comprend des parties de code de logiciel pour mettre en oeuvre les étapes du procédé selon l'une quelconque des revendications 1 à 8.

## FIG. 1

## FIG. 2

FIG. 3

200

NGS

DFS

PR1

PR2

PR3

2000

2002

FIG. 4

300

NGS

P

FIG. 5

FIG. 6

FIG. 7

F

SF

PR1_PR2

PR1_PR3

. . .

PR1_PRn

PR2_PR1

PR2_PR3

204

FIG. 8

EF

SF

PR1_PR2

PR1_PR3

206

# FIG. 9

1200

300

302

1202

RPCA

1208

304

1210

306

RFS

MF

1212

SF'(P_PRz)

400

Y

1214

N

200

1216

1218

# FIG. 10

302

PC

P

# FIG. 11

304

F

P_PR1

P_PRn

## FIG. 12

## FIG. 13

FIG. 14

1216

1230

VP (TP)

1232

Y

RP (TP)

1234    N

1236

1238

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1977237 B1
- EP 2392678 B1
- EP 2836837 B1
- EP 3237638 B1
- EP 2700038 B1

**Non-patent literature cited in the description**

- **TONG LI et al.** *Deep learning based feature-level integration of multi-omics data for breast cancer patients survival analysis,* 01 December 2020, vol. 20 (1
- **CHAI HUA et al.** Integrating multi-omics data through deep learning for accurate cancer prognosis prediction. *bioiv,* 06 May 2021
- **UNO HAJIME et al.** On the C-statistics for evaluating overall adequacy of risk prediction procedures with censored survival data. *STATISTICS IN MEDICINE,* 10 May 2011, vol. 30 (10), ISSN 0277-6715, 1105-1117
- Principal Component Analysis. **T. JOLLIFFE.** Springer Series in Statistics. Springer-Verlag, 2002
- Regression Shrinkage and Selection via the Lasso. **TIBSHIRANI, ROBERT.** Journal of the Royal Statistical Society, Series B (methodological). Wiley, 1996, vol. 58, 267-88
- **ABDULLAH, SITI et al.** Support Vector Machine, Multilayer Perceptron Neural Network, Bayes Net and k-Nearest Neighbor in Classifying Gender using Fingerprint Features. *International Journal of Computer Science and Information Security,* vol. 14 (7
- **THANH NOI, P. et al.** Comparison of Random Forest, k-Nearest Neighbor, and Support Vector Machine Classifiers for Land Cover Classification Using Sentinel-2 Imagery. *Sensors,* 2018, vol. 18, 18